Europäisches Patentamt

European Patent Office

Office européen des brevets

(19)

(11) Numéro de publication: **0 109 897**

**A1**

(12) # DEMANDE DE BREVET EUROPEEN

(21) Numéro de dépôt: **83402196.6**

(22) Date de dépôt: **15.11.83**

(51) Int. Cl.³: **A 61 M 3/00**
**A 61 M 7/00, A 61 M 29/02**

(30) Priorité: **17.11.82 FR 8219252**
**03.11.83 FR 8317472**

(43) Date de publication de la demande:
**30.05.84 Bulletin 84/22**

(84) Etats contractants désignés:
**AT BE CH DE GB IT LI LU NL SE**

(71) Demandeur: **David, Noel C., Dr.**
**15 Boulevard du Montparnasse**
**F-75006 Paris(FR)**

(72) Inventeur: **David, Noel C., Dr.**
**15 Boulevard du Montparnasse**
**F-75006 Paris(FR)**

(74) Mandataire: **Moulines, Pierre et al,**
**Cabinet BEAU de LOMENIE 55, rue d'Amsterdam**
**F-75008 Paris(FR)**

(54) **Perfectionnement aux sondes rectales pour lavements barytés.**

(57) Perfectionnement aux sondes rectales pour lavements barytés et qui comprennent une sonde ouverte à l'une de ses extrémités sur laquelle sont disposés deux balonnets gonflables dont l'un intra-rectal et l'autre extra-rectal.

Les deux ballonnets (4 et 6) qui sont situés à faible distance l'un de l'autre sur la sonde (1) communiquent entre eux afin d'être gonflés simultanément, de telle sorte que le sphincter (8) soit pris en tension douce entre les deux ballonnets (4 et 6).

L'invention est utilisée pour la fabrication de sondes rectales.

Fig. 1

EP 0 109 897 A1

1

## Perfectionnement aux sondes rectales pour lavements barytés.

La présente invention a pour objet un perfectionnement aux sondes rectales pour lavements barytés.

Pour procéder aux lavements barytés, il est connu d'utiliser des sondes du type Pouliquen munies d'un ballonnet introduit dans le rectum et rempli après son introduction par une certaine quantité d'eau ; ce ballonnet ayant pour but d'empêcher tout reflux à l'extérieur de l'index opaque, notamment du sulfate de baryum. Toutefois avec ce dispositif, il se produit certains accidents, en particulier une fissuration de la muqueuse de l'ampoule rectale, soit à la suite d'une effraction produite par l'extrémité de la sonde un peu trop rigide, soit par dilacération de la paroi en raison du gonflage intempestif chez certains sujets.

Pour remédier à cet inconvénient, on utilise un dispositif de sonde perfectionné suivant l'invention. Conformément à la présente invention, on utilise des sondes rectales pour lavements barytés qui comprennent une sonde ouverte à l'une de ses extrémités sur laquelle sont disposés deux ballonnets gonflables dont l'un intra-rectal et l'autre extra-rectal, caractérisées en ce que les deux ballonnets qui sont situés à faible distance l'un de l'autre sur la sonde communiquent entre eux afin d'être gonflés simultanément, de telle sorte que le sphincter soit pris en tension douce entre les deux ballonnets, le ballonnet intra-rectal étant situé à proximité de l'extrémité de la sonde présentant une ouverture.

Cette disposition permet d'obtenir une visualisation externe continue du remplissage des deux ballonnets, en raison de l'existence du ballonnet extra-rectal.

Par ailleurs, on obtient ainsi un meilleur effet d'obturation du sphincter anal qui est pris en tension douce entre les deux ballonnets.

Suivant une autre caractéristique de l'invention, le ballonnet intra-rectal présente une paroi en matériau déformable dont l'épaisseur est inférieure à celle de la paroi en matériau déformable constituant le ballonnet extra-rectal, de telle sorte que le gonflage du ballonnet intra-rectal s'effectue plus rapidement que celui du ballonnet extra-rectal.

Le dispositif suivant l'invention permet d'utiliser un ballonnet externe en communication avec le ballonnet interne et qui constitue un vase d'expansion capable d'amortir l'effet néfaste des ondes péristaltiques se produisant lors de l'instillation du sulfate de baryum ou de l'air en cas de pneumocolon associé.

Par ailleurs le ballonnet extérieur permet également de diminuer la pression intraluminale lors de l'injection du produit de contraste. Enfin un tel dispositif engendre des pressions compensées intra- et extra-rectales permettant d'éviter un certain nombre d'accidents dus à des ruptures iatrogènes, ruptures qui sont particulièrement fréquentes chez les sujets présentant des muqueuses fragilisées.

D'autres caractéristiques et avantages de l'invention seront mieux compris à la lecture de la description qui va suivre de plusieurs modes de réalisation et en se référant aux dessins annexés, sur lesquels;

- la figure 1 est une vue en coupe longitudinale d'une sonde rectale perfectionnée suivant l'invention.

- la figure 2 est une vue en coupe longitudinale d'un autre mode de réalisation d'une sonde.

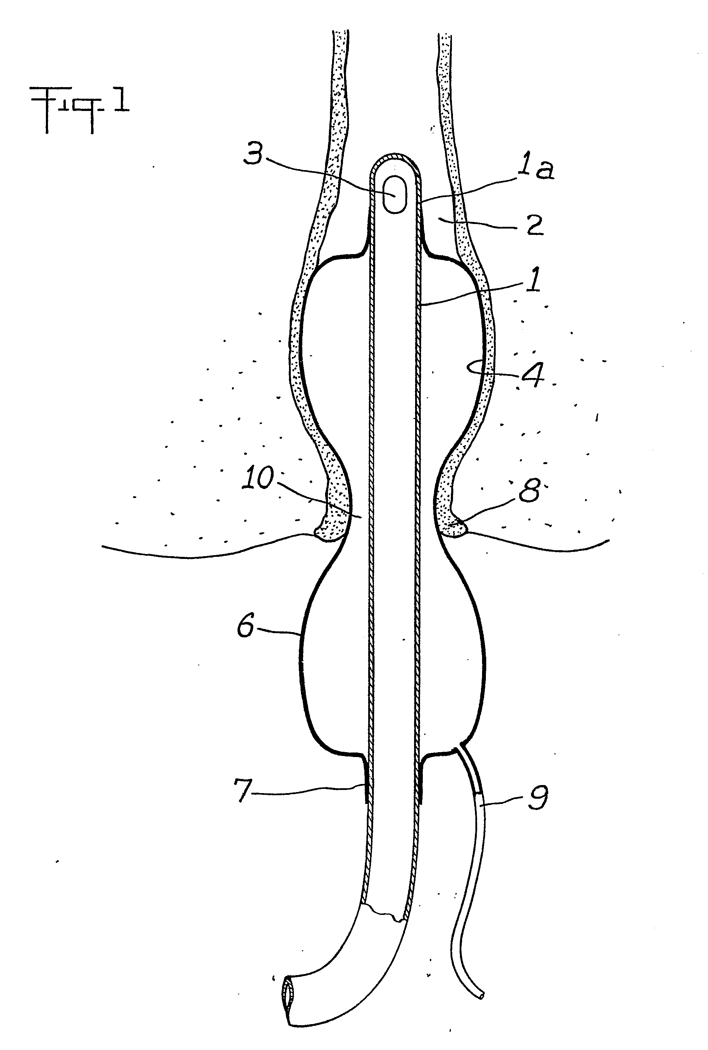

A la figure 1 on a représenté une sonde rectale 1 qui, à son extrémité engagée dans le rectum 2 du sujet, présente des ouvertures 3 pour le passage de l'index opaque, notamment un sulfate de baryum. A proximité de ladite extrémité 1a de la sonde, est disposé un ballonnet 4 qui est fixé par une partie tubulaire 5 sur la paroi de ladite sonde, ledit ballonnet 4 étant relié à un autre ballonnet 6 lui même fixé par une partie tubulaire 7 sur la sonde 1. Les ballonnets 4 et 6 qui communiquent entre eux afin d'être gonflés simultanément sont disposés sur la sonde de manière que le ballonnet 4 se trouve dans une position intra-rectale, alors que l'autre ballonnet 6 est dans une position extra-rectale.

De cette manière, le sphincter 8 du sujet est pris en tension douce entre les deux ballonnets 4 et 6. Le ballonnet extérieur 6 est relié à un conduit 9 d'insufflation à une source de liquide sous pression et il est prévu entre les ballonnets 4 et 6 un passage 10 qui permet la communication entre les deux ballonnets.

Sur le conduit d'insufflation 9, est monté un dispositif de mesure de pression du liquide non représenté au dessin.

L'extrémité la intra-rectale de la sonde est constituée d'un matériau présentant une grande élaset la longueur de cette partie de la sonde au-delà du ballonnet 4 est limitée au maximum.

Suivant l'invention, la sonde est introduite dans le corps du sujet, alors que les ballonnets 4 et 6 sont à létat dégonflé et qu'ils sont disposés comme représenté à la figure 1. On procède ensuite au gonflage des ballonnets 4 et 6 au moyen du conduit d'insufflation 9, ce qui permet de contrôler aisément le gonflage du ballonnet extérieur 6.

Afin d'éviter que la muqueuse ne puisse faire clapet au niveau de l'extrémité de la sonde, il est prévu deux ouvertures latérales situées de 0,5 à 1cm de l'extrémité distale de la sonde.

Toutefois on a constaté qu'il était préférable pour obtenir un remplissage convenable des ballonnets de procéder plus rapidement au gonflage du ballonnet intra-rectal et ensuite à l'équilibrage du gonflage entre les deux ballonnets lorsque le ballonnet intra-rectal a atteint un volume déterminé.

Cette disposition permet un meilleur maintien de la sonde.

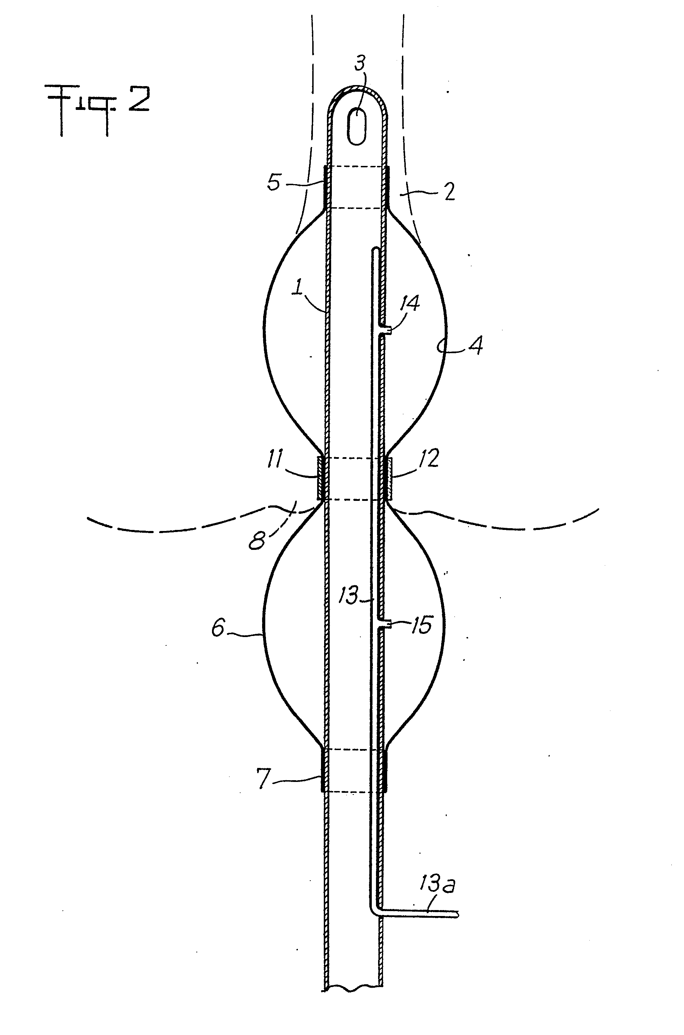

A la figure 2 on a représenté une sonde rectale 1 comme celle décrite ci-dessus et comportant à proximité de l'extrémité 1a, de la sonde un ballonnet 4 qui est fixé par une partie tubulaire 5 sur la paroi de ladite sonde, ledit ballonnet 4 étant relié à un autre ballonnet 6 lui même fixé par une partie tubulaire 7 sur la sonde 1.

La partie 11 reliant entre eux les ballonnets 4 et 6 est maintenue serrée contre la paroi de la sonde 1 au moyen d'une bague de serrage 12.

Toutefois la communication entre les ballonnets 4 et 6 est assurée au moyen d'un conduit 13 disposé à l'intérieur de la sonde 1 et qui débouche à travers la paroi de cette dernière par au moins un orifice 14 dans le ballonnet 4 et par au moins un orifice 15 dans le ballonnet 6.

Le conduit 13 qui assure l'équilibre entre les ballonnets est relié par sa partie 13a à une source de liquide ou de fluide sous pression afin de permettre le gonflage des ballonnets.

La paroi déformable du ballonnet 4 présente une épaisseur qui est inférieure à celle du ballonnet 6 de telle sorte que lors du gonflage de ceux-ci paar le conduit 13 le gonflage du ballonnet intra-rectal 4 s'effectue plus rapidement que celui du ballonnet extra-rectal 6.

Lorsque le ballonnet 4 atteint une certaine grosseur et pour une pression déterminée du liquide dans le ballonnet 4, il se produit un écoulement du liquide du ballonnet 4 vers le ballonnet 6 afin d'obtenir un équilibre de la pression du liquide dans les deux ballonnets et un gonflage identique en fin d'opération.

Pour privilégier le gonflage du ballonnet 4, il est également possible d'augmenter la section de

5

l'orifice 14 par rapport à l'orifice 15 ou d'utiliser plusieurs orifices 14 de même section par rapport à un seul orifice 15 de même section.

Il serait possible d'ajouter à la sonde et au canal d'instillation de la solution de contraste un conduit permettant de diriger de l'air de façon concomitante à l'index opaque.

De par sa conception cette canule grace à ses deux ballonnets communiquants, possède un embout intra-rectal très court ; donc le moins traumatisant possible dans un matériau souple.

Cette canule outre son utilisation pour les examens radiologiques du colon permet également de pratiquer, en minimisant les risques d'atteinte de la muqueuse rectale ou de rupture de la paroi colique, tout lavement évacuateur préalable.

Bien entendu l'invention n'est pas limitative et l'homme de l'art pourra y apporter des modifications sans sortir du domaine de l'invention.

R E V E N D I C A T I O N S

1.       Perfectionnement aux sondes rectales pour lavements barytés, et qui comprennent une sonde ouverte à l'une de ses extrémités sur laquelle sont disposées deux ballonnets gonflables dont l'un intra-rectal et l'autre extra-rectal, caractérisé en ce que les deux ballonnets (4 et 6) qui sont situés à faible distance l'un de l'autre sur la sonde (1) communiquent entre eux afin d'être gonflés simultanément, de telle sorte que le sphincter (8) soit pris en tension douce entre les deux ballonnets (4et 6) et en ce que le ballonnet intra-rectal (4) est situé à proximité de l'extrémité (1a) de la sonde (1) présentant une ouverture (3).

2.       Perfectionnement aux sondes rectales suivant la revendication 1 caractérisé en ce que le ballonnet intra-rectal (4) présente une paroi en matériau déformable dont l'épaisseur est inférieure à celle de la paroi en matériau déformable constituant le ballonnet extra-rectal (6) de telle sorte que le gonflage du ballonnet intra-rectal (4) s'effectue plus rapidement que celui du ballonnet extra-rectal (6).

3.       Perfectionnement aux sondes rectales suivant la revendication 2 caractérisé en ce que entre les deux ballonnets (4, 6) est disposée une bague de serrage (12) de la paroi (11) reliant les deux ballonnets et qui est maintenue en contact avec la paroi de la sonde (1).

4.       Perfectionnement aux sondes rectales suivant les revendications 2 et 3 caractérisé, en ce que les deux ballonnets (4, 6) sont en communication par un conduit (13) intérieur à la sonde (1) et débouchant dans chaque ballonnet (4, 6) par au moins un orifice (14, 15), ledit conduit (13) étant relié à une source de liquide sous pression.

5.       Perfectionnement aux sondes rectales suivant la revendication 4 caractérisé en ce que le conduit (13) d'amenée du fluide dans les deux ballonnets (4, 6) comporte au moins un orifice (14) dans le ballonnet intra-rectal (4) dont la section de passage est supérieure à la section de passage de l'orifice (15) débouchant dans le ballonnet extra-rectal (6).

6.            Perfectionnement aux sondes suivant la revendication 1, caractérisé en ce que le ballonnet extérieur (6) est relié par un conduit (9) d'insufflation à une source de liquide sous pression.

7.            Perfectionnement aux sondes suivant la revendication 1, caractérisé en ce que sur le conduit d'insufflation (9) est monté un dispositif de mesure de pression du liquide.

8.            Perfectionnement aux sondes suivant la revendication 1, caractérisé en ce que l'extrémité intra-rectale (1a) de la sonde (1) est constituée d'un matériau présentant une grande élasticité et dont la longueur située au-delà du ballonnet (4) est limitée au maximum.

9.            Perfectionnement aux sondes suivant la revendication 1, caractérisé en ce que deux ouvertures sont prévues latéralement de 0,5 à 1cm de l'extrémité distale de la sonde.

Fig 1

Fig.2

Office européen
des brevets

**RAPPORT DE RECHERCHE EUROPEENNE**

EP  83 40 2196

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | US-A-2 610 626  (J.D. EDWARDS) <br> * en entier * | 1,6,7 | A 61 M   3/00 <br> A 61 M   7/00 <br> A 61 M  29/02 |
| Y | | 9 | |
| | --- | | |
| X | FR-A- 582 423  (M. CHARNAUX) <br> * en entier * | 1,6,7 | |
| Y | | 9 | |
| | --- | | |
| Y | US-A-3 177 871  (P.H. MEYERS) <br> * en entier * | 9 | |
| | ----- | | |

| DOMAINES TECHNIQUES RECHERCHES (Int. Cl. ³) |
|---|
| A 61 M |

Le présent rapport de recherche a été établi pour toutes les revendications

| Lieu de la recherche <br> LA HAYE | Date d'achèvement de la recherche <br> 26-01-1984 | Examinateur <br> FERGUSON J.R. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82